# EUROPEAN PATENT APPLICATION

(11) **EP 3 132 818 A1**
(43) Date of publication of application: **22.02.2017**
(21) Application number: 16178228.9
(22) Date of filing: 15.12.2005
(51) Int. Cl.: A61M 5/00, A61M 1/36, A61B 17/12, A61B 17/135, A61B 17/11, A61B 17/00, A61M 25/10, A61M 39/02, A61M 39/28

(54) **METHOD OF TREATING COPD WITH ARTIFICIAL ARTERIO-VENOUS FISTULA AND FLOW MEDIATING SYSTEMS**

(30) Priority: 15.12.2004 US 13981
(62) Divisional of application: 05854613.6
(71) Applicant: Rox Medical, Inc., Carlsbad, CA 92008 (US)
(72) Inventor: BRENNEMAN, Rodney A., San Juan Capistrano, CA 92675 (US); FLAHERTY, J. Christopher, Topsfield, MA 01983 (US)
(74) Representative: Price, Nigel John King

(57) **Abstract**

A method for treatment of COPD, hypertension, and left ventricular hypertrophy, and chronic hypoxia including creation of an artificial arterio-venous fistula and installation of a flow mediating device proximate the fistula. The flow mediating device is operated to limit flow as medically indicated to provide the optimum amount of bypass flow.

## Description

### Field of the Inventions

The inventions described below relate to treatments for pulmonary hypertension and vascular surgery.

### Background of the Inventions

Chronic obstructive pulmonary disease (COPD), chronic hypoxia, hypertension, and left ventricular hypertrophy and pulmonary hypertension are diseases of the cardiopulmonary system. Chronic obstructive pulmonary disease (COPD), which includes chronic bronchitis and emphysema, is a slowly progressive lung disease caused primarily by smoking. In COPD, the lungs are damaged and the airways are partly obstructed, making it difficult to breath and leading to a gradual loss of lung function. Symptoms of COPD include chronic cough, excessive sputum production, low blood oxygen levels and severe disabling shortness of breath. COPD represents the fourth leading cause of death in the United States. Chronic hypoxia (reduction of oxygen supply to the body despite adequate blood flow through the body), hypertension, and left ventricular hypertrophy are related conditions which may be symptomatic of COPD or coincident with COPD.

These serious conditions affect many people, and the primary treatments are merely ameliorative. The primary treatments for COPD include avoidance of irritants such as tobacco smoke and breathing supplemental oxygen. In advanced cases of COPD, lung reduction surgery is sometimes performed, but it is not clear that it helps. There is no known cure for COPD.

An aortocaval fistula (ACF) is a rare clinical condition that can be either spontaneous (80% of the cases), related to abdominal aortic aneurysm, or the result of some trauma such as lumbar disk surgery. It is currently seen as a defect that should be cured with surgery and, possibly, stent-graft implantation in the aorta. Likewise, arterio-venous fistulas are uncommon, and can be caused by trauma or may be iatrogenic (i.e.., an unintended result of vascular intervention, as discussed in Ruebben, et al., Arteriovenous fistulas induced by femoral arterial catheterization: percutaneous treatment, 209 Radiology, 729 (1998)). Arteriovenous fistulas are also see as defects that should be cured with surgery and, possibly, stent-graft implantation.

Contrary to this understanding, an intentionally formed aortocaval fistula appears to be a viable treatment for COPD. Recently, in our co-pending U.S. Patent Application 10/820,169 (Attorney Docket Number S03-013/US) filed April 6, 2004, entitled Implantable Arteriovenous Shunt Device and listing John L. Faul, Toshihiko Nishimura, Peter N. Kao & Ronald G. Pearl as inventors (the entirety of which is hereby incorporated by reference), we propose creation of an artificial aortocaval fistula as a treatment for COPD, and we disclose the method of creating the fistula and an implantable shunt for maintaining the aortocaval fistula. In our co-pending U.S. Patent Application 10/927,704 filed August 27, 2004 (the entirety of which is hereby incorporated by reference) we disclose a vascular shunt rivet which serves to hold contiguous points of the patient's aorta and inferior vena cava (or other arteries and their associated veins, such as the femoral artery and femoral vein, or the carotid artery and the carotid vein) together and maintain an open flow path from the aorta to the vena cava. The device functions as a rivet, holding the two vessel walls in close proximity, and as a shunt, permitting and maintaining flow from one blood vessel to the other as a treatment for COPD.

The method of treating COPD by creating an artificial arterio-venous fistula and maintaining the fistula with an endoprosthesis may be improved with the addition of mechanisms for adjusting the arterial bypass flow rate to optimum levels. Adjustments to flow may be made to balance the positive effects of injecting oxygenated blood into the venous system with the potential negative effects.

### Summary

The devices and methods described below provide for treatment of COPD, hypertension, and left ventricular hypertrophy, and chronic hypoxia. After creation of an artificial arterio-venous fistula, a flow mediating device is installed proximate the fistula (directly on the fistula, immediately upstream of the fistula in the artery, or immediately downstream of the fistula in the vein). In one embodiment of the method, a bladder is installed proximate the vein, artery or the fistula itself, and is inflated to impinge upon the vein, artery or the fistula to limit bypass flow through the fistula. Other mechanisms for controlling flow are also proposed, including shunts (placed to maintain the fistula) having mechanisms for throttling flow through the shunt, and even prior art screw operated clamps for compressing some portion of the flow path.

### Brief Description of The Drawings

Figure 1 illustrates the method of creating an artificial arterio-venous fistula and controlling flow with a balloon impinging on the artery.
Figure 2 is a detail view of the inflatable cuff and its associated components, installed over the femoral vein of the patient.
Figure 3 illustrates installation of the cuff over the left femoral artery.
Figures 4, 5 and 6 illustrate use of a bladder which merely impinges on the femoral vein to control bypass flow.
Figures 7 and 8 illustrates a device and method for percutaneous insertion of a bladder to a subcutaneous space adjacent the blood vessel which has been modified with the creation of the arterio-venous fistula.
Figure 9 illustrates placement of the implantable self-sealing bladder of Figure 7 proximate a vein so as to impinge on the vein to inhibit flow through a nearby arterio-venous fistula.
Figure 10 illustrates placement of the implantable self-sealing bladder of Figure 7 proximate an artery so as to impinge on the artery to inhibit flow through a nearby arterio-venous fistula.
Figures 11 and 12 illustrate a bladder assembly which assists in operably coupling the bladder to the blood vessel.
Figure 13 shows the bladder assembly of Figure 12 disposed in impinging relationship with the anatomical fistula.
Figures 14 and 15 illustrate use of shunt with an integral bladder, which may be inflated to control flow through the shunt, as desired to affect the arterial bypass flow as treatment for COPD.
Figures 16 and 17 illustrate another mechanism for regulating fistula bypass flow, in which a membranous wall of the shunt may be magnetically drawn to control the size of the shunt lumen.

### Detailed Description of the Inventions

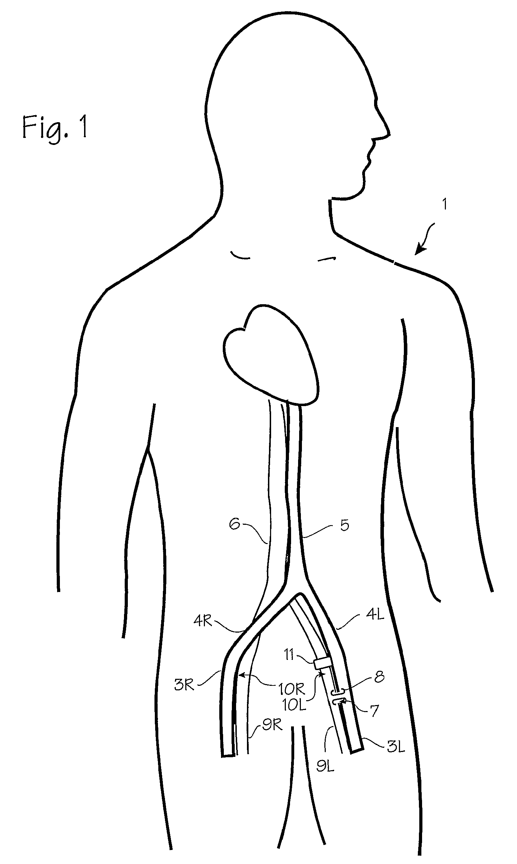

Figure 1 illustrates the method of creating an artificial arterio-venous fistula and controlling flow with a balloon impinging on the artery. A portion of the vasculature of the patient **1** is shown, illustrating the left and right femoral artery/external femoral arteries **3**L and 3R, the left and right common iliac arteries 4L and 4R, and the abdominal aorta **5.** Portions of the venous system are also illustrated, including the vena cava **6,** which runs parallel to the aorta, and is typically contiguous with the aorta, and the left and right femoral veins 9L and 9R. An artificial arterio-venous fistula or side-to-side anastomosis **7** may be formed between the femoral vein and femoral artery on either side of the body, indicated as items **10R** and **10L,** or between the iliac artery and the femoral vein, and at locations within the aorta. The artificial fistula may be maintained as an anatomical fistula, consisting of vascular tissue, if the local anatomy tends to heal to a stable and patent fistula, or it may be maintained by shunt or shunt rivet 8 as illustrated, or by an endoprosthesis (a vascular graft or stent graft) of significant length.

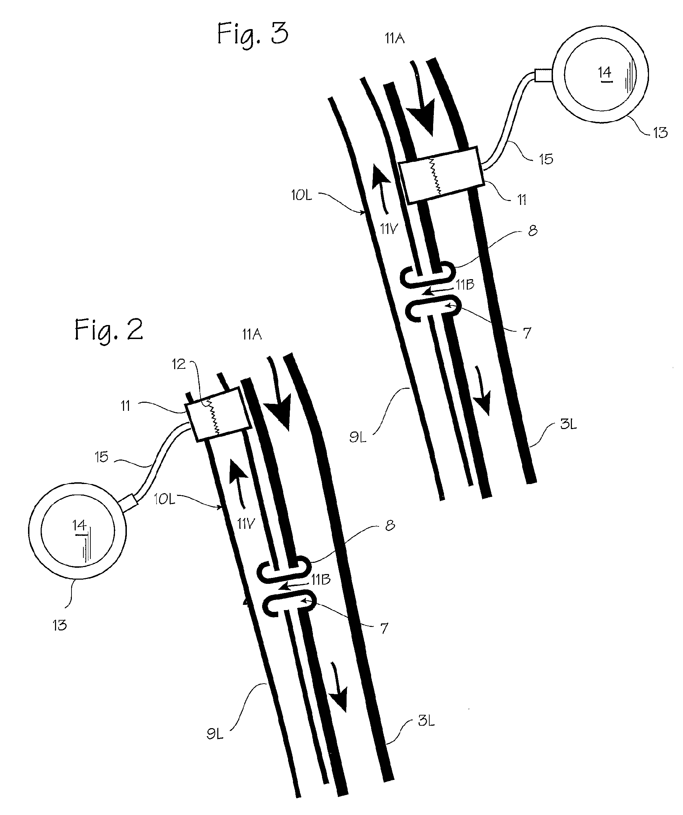

To regulate flow through the fistula, an inflatable cuff **11** is placed and implanted around the femoral vein, proximal to the fistula (closer to the heart relative to the fistula). The inflatable cuff is further illustrated in Figure 2, which shows the inflatable cuff assembly which includes the cuff 11, secured around the vein with suture seam **12,** a subcutaneous injection port **13** with a resealable membrane **14,** and a short conduit **15** providing for fluid communication between the injection port and the cuff (the injection port and resealable membrane may also be formed integrally with the cuff). The cuff may also be installed over the femoral artery 3L, proximal to the fistula, as shown in Figure 3. Inflation of the cuff results squeezing the blood vessel within the cuff, essentially throttling flow through the blood vessel. The degree to which flow is mediated or throttled depends on the degree to which the cuff is inflated.

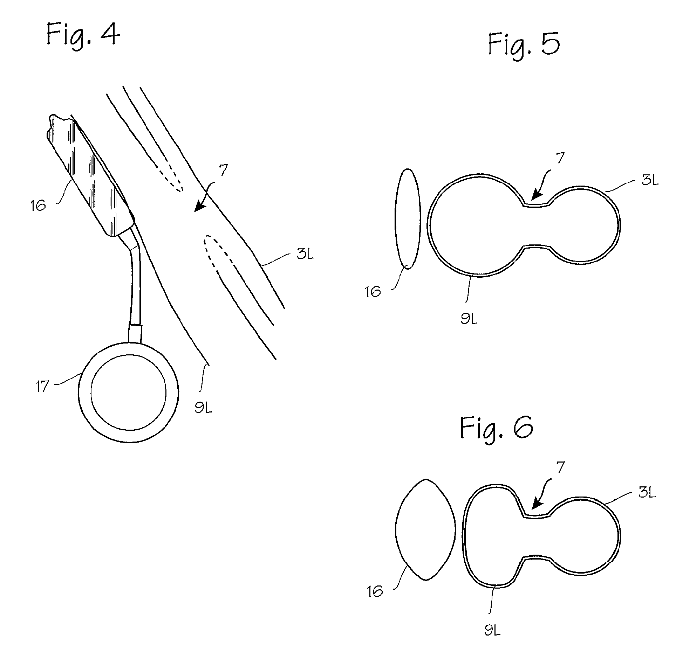

Figures 4, 5 and 6 illustrate use of bladder which merely impinges on the femoral vein to control bypass flow. As shown in Figure 4, a bladder is placed in immediate contact with the femoral vein 9L. The fistula 7 is shown in phantom, and may be fitted with a shunt or rivet 8 as shown in Figures 1 through 3. The bladder **16** is an elongate bladder, which may be conformal or non-conformal, which is inflated through the associated access port **17.** Figure 5 shows a cross section of the leg, with the bladder uninflated, impinging on the femoral vein, while Figure 6 illustrates the effect of the inflated bladder on the femoral vein 9L. Upon inflation, the bladder further impinges upon the femoral vein to impede flow, and thereby impede bypass flow from the femoral artery to the femoral vein.

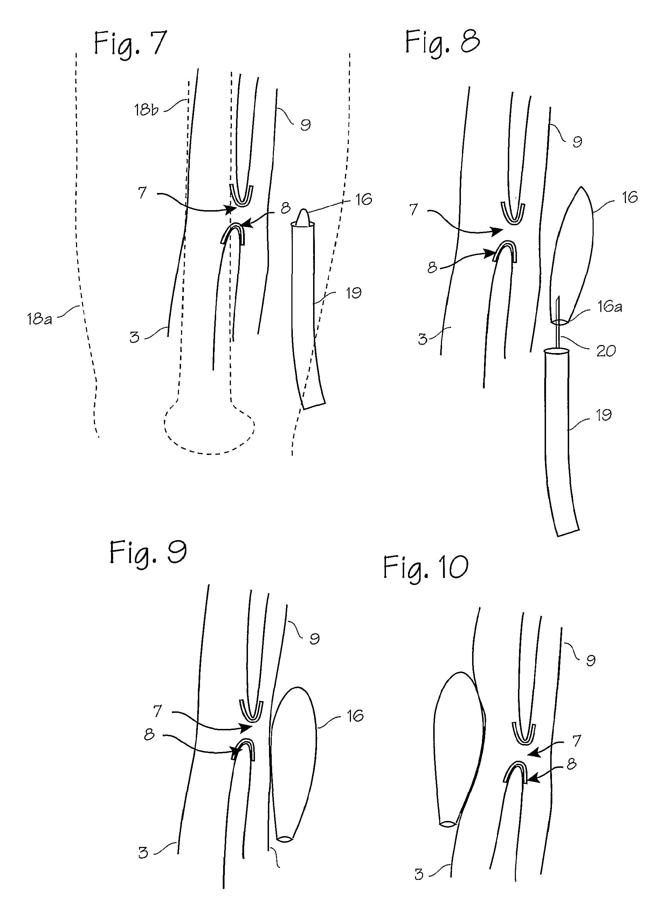

Figures 7 through 10 illustrate a device and method for percutaneous insertion of a bladder to a subcutaneous space adjacent the blood vessel which has been modified with the creation of the arterio-venous fistula 7 and, optionally, installation of the shunt rivet 8. This method may be beneficially employed, especially where the artery/vein pair is located proximate a rigid anatomic structure. In Figure 7, the artery/vein pair is the femoral artery 3 and the femoral vein 9, shown within the thigh **18**a of a patient, with the femur 18b shown in phantom. A bladder 16 is housed within the insertion catheter **19.** The insertion catheter is inserted percutaneously, and pushed through the body tissue as necessary to locate the bladder near the arterio-venous fistula 7. As shown in Figure 8, the insertion catheter is withdrawn to expose the bladder and release it proximate the femoral vein 9, and a needle **20** is delivered through the insertion tube and pushed through a self-sealing wall portion 16a of the bladder so that the bladder may be inflated as desired. The self-sealing wall portion may be a discrete section of the bladder comprises a thick silicone membrane or the like, which may be pierced with a typical hypodermic needle but resiliently seal any punctures by virtue of the resiliency of the membrane. The entire bladder may be made of suitable self-sealing material, so that a surgeon may inflate it by injection fluid at any point on the bladder.

The bladder may be positioned proximal to the fistula or distal to the fistula, and may be positioned to impinge of the artery or vein, and may be placed anywhere around the blood vessels, though positioning superiorly to a nearby rigid anatomical structure, with the blood vessel trapped between the bladder and the anatomical structure may be preferred to ensure that the blood vessel is compressed rather than merely displaced by the bladder. Figure 9 illustrates placement of the implantable self-sealing bladder proximate the femoral vein so as to impinge on the femoral vein to inhibit flow through a nearby arterio-venous fistula. The bladder may also be place in proximity to the femoral artery, and Figure 10 illustrates placement of the implantable self-sealing bladder proximate the femoral artery so as to impinge on the femoral artery to inhibit flow through a the arterio-venous fistula. With the bladder in place, a surgeon may access the bladder by inserting a needle through the patients skin, with or without the aid of an insertion catheter, and inflate or deflate the bladder as desired.

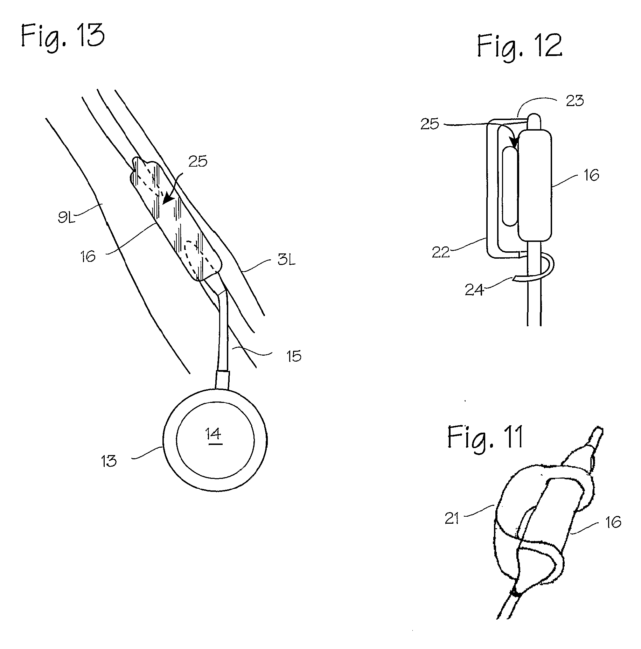

Figures 11 and 12 illustrate a bladder assembly which assists in operably coupling the bladder to the blood vessel, so that distention of the bladder is certain to result in impingement on the blood vessel. In Figure 11, the bladder 16 is coupled to a band **21** which provides an anvil against which the balloon pushes and compresses the blood vessel, and prevents the blood vessel from merely moving in response to bladder inflation. The band may be attached to the balloon at each end, as shown, or the band may be wrapped completely around both the bladder and the blood vessel. Figure 12 illustrates another a bladder assembly which assists in operably coupling the bladder 16 to the blood vessel, so that distention of the bladder is certain to result in impingement on the blood vessel. In this figure, a relatively hard and rigid clip **22** is hinged or otherwise rotatably attached to the balloon at hinge point **23,** on one end or the other, and is fastened with the hook or other closure mechanism **24** at the other, so that the bladder may be fastened to the blood vessel. The clip is narrow and elongate, so that it may be used as shown in Figure 13, with the bladder 16 disposed in impinging relationship with the anatomical fistula **25** and the clip disposed on the opposite side of the fistula and closed upon the bladder or an extending structure (in this case, the conduit used to fill the bladder). If a graft of significant length is used, the devices of Figures 11, 12 and 13 may be placed over contiguous parallel segments of the shunt and artery, or the shunt and the vein. Each of the bladder devices disclosed may comprise self-sealing walls, in a portion or over substantially the entire bladder surface, so that the bladder may be injected with fluid by merely piercing the bladder itself with a hypodermic needle, injecting fluid, and removing the needle.

Any other adjustable vascular impingement device may be used, including the Flow-watch® pulmonary artery band system which includes a jack screw adjusted by a motor which is powered and controlled telemetrically, as described in Stergiopulis, Flow Control Device and Method, PCT App. PCT/EP00/06907 (Jan. 25, 2001), or screw operated bands such as those disclosed in Schlensak, et al., Pulmonary Artery Banding With A Novel Percutaneously, Bidirectionally Adjustable Device, 12 Eur. J. of Cardio-thoracic Surg. 931-933 (1997).

The devices and methods described above may be used to treat COPD as follows. First, a surgeon creates a fistula between an artery and a nearby vein. Preferably, the artery and vein are large, such as the femoral artery and the femoral vein. The fistula may be maintained, after artificial creation, either naturally to create an anatomical fistula comprising portions of the contiguous artery and vein healed together, or it may be a mechanically maintained fistula which is supported with a shunt or stent, or it may comprise a distinct shunt from the artery to the vein. After creating and stabilizing the fistula (ensuring that endoprosthesis are securely implanted, or that the anatomical fistula is structurally sound), the surgeon implants the flow restricting device (which may be any one of the devices described or mentioned herein) around the vein downstream from the fistula, or around the artery upstream from the fistula, or across the fistula itself. To control flow through the fistula, the cuff is inflated or deflated as necessary to achieve a desired bypass flow volume. The desired by-pass flow volume is determined by monitoring blood oxygenation and cardiac function intra-operatively (that is, immediately after creation of the fistula and implantation of the flow restricting device) and/or (that is, before discharge) and adjusting bypass flow to obtain a medically indicated short-term change in such parameters. The desired by-pass flow should also be determined and adjusted post-operatively, after a stabilization period (a few weeks after surgery). The shunt will increase mixed venous oxygenation, (SvO2), increase the percentage of oxygen bound to hemoglobin (SpO2), increase the amount of oxygen dissolved in blood plasma (PaO2), and increase cardiac output and stroke volume (after remodeling). Initially (immediately after opening the shunt) the heart rate increases to provide increased cardiac output. Then, as the heart 'remodels' the stroke volume increases and the heart rate comes back down to normal levels to maintain increased cardiac output. Lower bypass flow in the post-operative and stabilization time period may be desirable to avoid over stressing the heart and allow a more gradual cardiac remodeling. Thus, the overall procedure may be accomplished by adjusting flow in the peri-operative and stabilization time frame to levels sufficient to increase PaO₂ and/or SvO₂ about 5% or more, and increase cardiac output by about 10% or more, followed by re-evaluation of the patient after stabilization and readjustment of by-pass flow to provide for an increase PaO₂ and/or SvO₂ (relative to pre-operative levels) of about 10% to 20% or more, depending on patient tolerance. Should the heart rate increase attendant to the bypass flow be more tolerable, the bypass flow in the peri-operative and stabilization time frame may adjusted to higher levels, to provide for an increase in PaO₂ and/or SvO₂ of about 20% to 25% (for a COPD with low PaO₂ and/or SvO₂), followed by re-evaluation of the patient after stabilization (after long-term remodeling of the heart, the heart may be remodeled in response to the therapy) and reduction of by-pass flow to provide for an increase PaO₂ and/or SvO₂ (relative to pre-operative levels) by about 10% to 20%. The optimal levels of these parameters, and the optimum trade-off between increased blood levels, cardiac output and increased heart rate are expected to be refined with clinical experience.

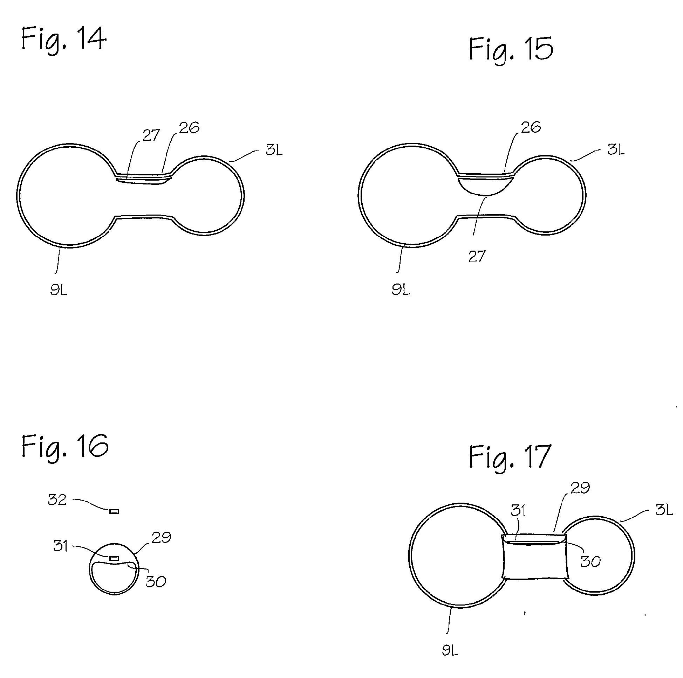

Rather than impinging on the blood vessel as described above, the desired flow control may be achieved by providing a shunt with a variable lumen cross-section or other flow control means which may act as a throttle valve. Figures 14 and 15 illustrate use of shunt with an integral bladder which may be inflated to control flow through the shunt, as desired to effect the arterial bypass flow as treatment for COPD. A shunt **26** is installed between the femoral artery and the femoral vein. The shunt additionally comprises a bladder **27** installed within the lumen of the shunt, which is filled as desired through the inflation port **28.** As illustrated in Figure 15, in which the bladder is partially inflated, the bladder partially occludes the shunt, to a degree dependent on the degree to which the bladder is inflated. The bladder may be fully inflated to fully occlude the shunt and prevent bypass flow. The shunt may be made of any suitable shunt material.

Figures 16 and 17 illustrate another mechanism for regulating fistula bypass flow, in which a membranous wall of the shunt may be magnetically drawn to control the size of the shunt lumen. In this embodiment, the shunt is provided with an rigid outer wall **29** and flexible inner wall **30.** The dissectible portion of the inner wall which cuts across the lumen may be elastic or merely loose, so that it may be pulled against the outer wall to fully open the lumen. A magnet (or ferromagnetic mass) **31** is fixed to the dissectible portion of the inner wall, such that the magnet, and thus the dissectible portion of the inner wall, may be drawn against the outer by magnetic attraction to an extracorporeal magnet **32.** The extracorporeal magnet may be an electromagnet with operating circuitry which is fixed to the patient in proximity to the shunt, or it may be a permanent magnet, the power of which may be selected to effect a desired degree of openness. Because the inner wall is flexible, the opening effect of the magnet depends on its proximity to the magnet 31. When the extracorporeal magnet 32 is removed from a position proximate the magnet 31, the membrane reverts to its original position, which in Figure 16 is partially occluded, and when the extracorporeal magnet 32 is placed in proximity to the magnet 31, the membrane is drawn tight to the rigid outer wall to open the lumen. The operation can be reversed by forming the flexible inner wall to closely adhere to the rigid outer wall when relaxed, such that the magnet may be used to pull or push the membrane away from the rigid outer wall to occlude the lumen. As an alternative to the flexible membrane, the inner wall may comprise a tube with a plastically deformable portion that may be pulled, by operation of the magnet 31 secured to the deformable portion of the tube and the extracorporeal magnet, to establish the desired lumen size.

While the devices and methods have been described relative to the femoral artery and femoral vein, they may also be employed in other suitable contiguous or associated artery/vein pairs, including the aorta and inferior vena cava, the femoral artery and the iliopopliteal vein or iliac vein, the popliteal artery and popliteal vein, the carotid artery and the jugular vein, the brachial artery and brachial vein, and the brachio-cephallic artery and subclavian vein. The artery-to-vein shunt may also be provided between remote anastomosis cites, such as the iliac artery to the inferior vena cava. Also, though discussed in terms of COPD treatment, the method should be useful to treat hypertension (pulmonary hypertension and arterial hypertension), left ventricular hypertrophy, and chronic hypoxia. Thus, while the preferred embodiments of the devices and methods have been described in reference to the environment in which they were developed, they are merely illustrative of the principles of the inventions. Other embodiments and configurations may be devised without departing from the spirit of the inventions and the scope of the appended claims.

The claims of the parent application are reproduced below on pages 14-18. These clauses define preferred embodiments. The applicant reserves the right to pursue protection for the combinations of features set out in these clauses, and/or for any other subject-matter contained in the parent application as filed, either in the present divisional application or in a further application divided from the present divisional application. The claims of the parent application are not the claims of this divisional application.

### Preferred Embodiments

1. A system for controlling flow through an artificial arterio-venous fistula in a patient, wherein said arteriovenous fistula communicates between an artery and a vein of the patient, said system comprising:
   a bladder adapted for implantation into the body of a patient proximate the arteriovenous fistula;
   means for percutaneously inserting the bladder into the body and positioning the bladder proximate the artery or vein and the location of the arterio-venous fistula;
   means for injecting fluid into the bladder.
2. The system of clause 1 wherein the bladder comprises a self-sealing wall portion, said self-sealing wall portion being adapted to permit passage of the means for injection fluid.
3. The system of clause 1 wherein substantially the entire bladder comprises a self-sealing material, said self-sealing material being adapted to permit passage of the means for injection fluid.
4. The system of clause 1 further comprising a band, operably connected to the bladder and adapted to surround the fistula, artery or vein, and secure the bladder to the fistula, artery or vein proximate the arterio-venous fistula.
5. The system of clause 1 further comprising a rigid fastener, operably connected to the bladder and adapted to surround the fistula, artery or vein, and secure the bladder to the fistula, artery or vein proximate the arterio-venous fistula.
6. A flow control device for mediating flow through an arterio-venous fistula between an artery and vein in a patient, said flow control device comprising:
   a shunt or graft adapted to connect an artery to a vein, said shunt or graft having a lumen extending therethrough;
   throttling means operable to mediate flow through the shunt or graft.
7. The flow control device of clause 6 wherein the throttling means comprises a dissectible wall portion within the lumen, a magnetic mass fixed to the dissectible wall portion, said magnetic mass being operable to deflect the dissectible wall portion when acted upon by a magnet.
8. The flow control device of clause 6 wherein the throttling means comprises a bladder disposed within the lumen, and inflating the bladder as desired to occlude the lumen to the degree necessary to obtain desired bypass flow.
9. The flow control device of clause 6 wherein the throttling means comprises compliant sidewalls on the shunt or graft and a piston disposed outside the shunt or graft and means for driving the piston against the sidewall of the shunt or graft to squeeze the shunt or graft to change the size of the lumen.
10. The flow control valve of clause 8 wherein the bladder further comprises a self-sealing material.
11. A method of treating COPD in a patient, said method comprising the steps of:
   creating an artificial fistula between an artery and a vein of the patient; installing a flow mediating device proximate the artificial fistula;
   operating the flow-mediating device to control bypass blood flow through the fistula as indicated to treat COPD.
12. The method of clause 1 further comprising:
   operating the flow-mediating device to control bypass blood flow through the fistula to achieve an increase in the patient's Pa02 by at least about 5% as indicated to treat COPD.
13. The method of clause 1 further comprising:
   operating the flow-mediating device to control bypass blood flow through the fistula to achieve an increase in the patient's Sv02 by at least about 5% as indicated to treat COPD.
14. The method of clause 1 further comprising:
   operating the flow-mediating device to control bypass blood flow through the fistula to achieve an increase in the patient's cardiac output by at least about 10% as indicated to treat COPD.
15. The method of clause 1 further comprising:
   evaluating the patient after a stabilization period and adjusting the flow mediating device to control bypass blood flow through the fistula to achieve an increase in the patient's cardiac output by at least about 10%.
16. The method of clause 1 further comprising:
   providing the flow control device in the form of an inflatable bladder system comprising an inflatable cuff adapted to substantially surround a portion of the patient's vasculature proximate the fistula and .means for delivering fluid to the inflatable cuff.
17. The method of clause 1 further comprising:
   providing the flow control device in the form of an inflatable bladder adapted to impinge upon a portion of the patient's vasculature proximate the fistula and means for delivering fluid to the inflatable bladder.
18. The method of clause 1 further comprising the steps:
   of providing a band adapted to surround a vessel of the patients vasculature and securing the bladder to the patients vasculature with the band, proximate the fistula.
19. The method of clause 1 further comprising:
   providing the flow control device in the form of an inflatable bladder adapted to impinge upon a portion of the patient's vasculature proximate the fistula, rigid fastener operably connected to the bladder and means for delivering fluid to the inflatable bladder; and
   installing the bladder against a portion of the patient's vasculature proximate the fistula and securing the bladder against the portion of the patient's vasculature by placing a portion of the rigid fastener opposite the bladder with the portion of the patient's vasculature disposed between the bladder and the rigid fastener, and securing the rigid fastener to the bladder.
20. The method of clause 1 further comprising:
   providing the flow control device in the form of a shunt or graft between an artery and a nearby vein, and providing throttling means operable to mediate flow through the shunt of graft.
21. The method of clause 10 further comprising:
   providing the throttling means in the form of a shunt or graft having a lumen and a bladder disposed within the lumen, and inflating the bladder as desired to occlude the lumen to the degree necessary to obtain desired bypass flow.
22. The method of clause 10 further comprising:
   providing the throttling means in the form of a shunt or graft having a lumen and a dissectible wall portion within the lumen, a magnetic mass fixed to the dissectible wall portion, said magnetic mass being operable to deflect the dissectible wall portion when acted upon by a magnet.
23. The method of clause 10 further comprising:
   providing the throttling means in the form of a shunt or graft having a lumen and compliant sidewalls and a piston disposed outside the shunt or graft and means for driving the piston against the sidewall of the shunt or graft to squeeze the shunt or graft to change the size of the lumen; and
      operating the piston to squeeze or release the shunt or graft as desired to mediate flow through the shunt or graft.
24. The method of clause 1 further comprising: placing the flow mediating device on the vein downstream from the fistula.
25. The method of clause 1 further comprising:
   placing the flow mediating device on the artery upstream from the fistula.
26. The method of clause 1 further comprising:
   placing the flow mediating device directly over the fistula.
27. The method of clause 1 further comprising:
   creating the artificial fistula between a femoral artery and femoral vein of the patient.
28. The method of clause 1 further comprising:
   creating the artificial fistula between one of the following vein/artery pairs:
      the aorta and inferior vena cava, the femoral artery and the iliopopliteal vein or iliac vein, the carotid artery and the carotid vein or jugular vein, the brachial artery and brachial vein, and the brachio-cephallic artery and subclavian vein.

## Claims

1. A flow control device for mediating flow through an arterio-venous fistula between an artery and a vein in a patient, the flow control device comprising:
a shunt adapted to connect the artery to the vein, the shunt comprising:
a lumen extending therethrough; and
a flow mediating device positioned within the lumen of the shunt,
wherein the flow mediating device comprises a dissectible wall portion within the lumen of the shunt and a magnetic mass positioned within the dissectible wall portion,
wherein the magnetic mass is made operable to deflect the dissectible wall portion when acted upon by a magnet to mediate flow through the shunt when the flow mediating device is in use.

2. The flow control device of claim 1, wherein the shunt comprises a rigid outer wall and a flexible inner wall, wherein the dissectible wall portion is a portion of the flexible inner wall.

3. The flow control device of claim 1, wherein the magnetic mass is fixed to the dissectible wall portion.

4. The flow control device of claim 1, wherein the magnet is an electromagnet with operating circuitry in proximity to the shunt.

5. The flow control device of claim 1, wherein the magnet is a permanent magnet.

6. The flow control device of claim 1, wherein the shunt connects the artery to the vein in a side-to-side anastomosis configuration.

7. The flow control device of claim 1, wherein the shunt is a shunt rivet.

8. The flow control device of claim 1, wherein the artery is a femoral artery and the vein is a femoral vein.

9. The flow control device of claim 1, wherein the artery is the aorta and the vein is the inferior vena cava.

10. The flow control device of claim 1, wherein the artery is the carotid artery and the vein is the jugular vein.

11. The flow control device of claim 1, wherein the artery is the iliac artery and the inferior vena cava.
